# DEMANDE DE BREVET EUROPEEN

(11) **EP 4 716 434 A1**
(43) Date de publication de la demande: **25.03.2026**
(21) Numéro de dépôt: 25202464.1
(22) Date de dépôt: 16.09.2025
(51) Int. Cl.: H10K 30/87, H10K 39/30, A61B 5/1455

(54) **DISPOSITIF PHOTODETECTEUR A ELEMENT DE GUIDAGE OPTIQUE**

(30) Priorité: 24.09.2024 FR 2410191
(71) Demandeur: Commissariat à l'Energie Atomique et aux Energies Alternatives, 75015 Paris (FR)
(72) Inventeur: RACINE, Benoît, 38054 GRENOBLE CEDEX 09 (FR); BLANQUER, Guillaume, 38054 GRENOBLE CEDEX 09 (FR); PLANAT-CHRETIEN, Anne, 38054 GRENOBLE CEDEX 09 (FR); LAUGIER, Christelle, 38054 GRENOBLE CEDEX 09 (FR)
(74) Mandataire: Cabinet Beaumont

(57) **Abrégé**

La présente description concerne dispositif photodétecteur (100) comprenant :
- un élément de guidage optique (104) ;
- un composant électronique (102) de conversion de lumière en un signal électrique, configuré pour détecter de la lumière du côté d'une première face (116) du composant électronique disposée en regard de l'élément de guidage optique et du côté d'une deuxième face (118) du composant électronique opposée à la première face ;
- une couche réflectrice (120) disposée du côté de la deuxième face du composant électronique ;
- un composant optique (124) disposé entre la couche réflectrice et l'élément de guidage optique et au moins partiellement transparent à la lumière destinée à être détectée par le composant électronique ;
et dans lequel la couche réflectrice forme une surface réfléchissante (122) conforme à une surface non plane du composant optique sur laquelle la couche réflectrice est disposée.

## Description

### Domaine technique

La présente description concerne de façon générale les dispositifs photodétecteurs, ou photorécepteurs, à éléments de guidage optique, ou éléments de guidage lumineux.

### Technique antérieure

La lumière est une modalité de mesure de plus en plus utilisée dans le domaine de la santé pour déterminer, ou diagnostiquer, et suivre, ou monitorer, des paramètres physiologiques d'intérêt telles que le rythme cardiaque, le taux d'oxygénation du sang ou de glycémie des personnes. Pour cela, de la lumière est injectée à travers l'épiderme puis collectée après avoir interagi par des processus d'absorption et/ou de diffusion avec les tissus d'intérêts, permettant de remonter aux paramètres physiologiques ciblés. La lumière collectée est toutefois très faible à cause du fort taux d'absorption et de diffusion de la lumière dans les tissus.

Il est connu d'utiliser des photodiodes organiques, ou OPD (« Organic Photodiode » en anglais) pour recevoir et capter de la lumière, et la transformer en une grandeur mesurable correspondant à un signal électrique. Les OPDs ont atteint aujourd'hui des performances qui permettent leur utilisation dans différents produits et domaines d'application. L'utilisation de matériaux organiques pour réaliser une photodétection présente plusieurs avantages par rapport aux photodiodes semi-conductrices : accès à toutes les propriétés intrinsèques de ces matériaux, à la géométrie de l'OPD et aux longueurs d'onde d'absorption, possibilité de dépôt sur des supports souples permettant par exemple un ajustement optimal de l'OPD sur une partie du corps pour des applications médicales.

Malgré ces différents avantages, les OPDs sont limitées dans leur utilisation à cause de leur courant d'obscurité élevé (supérieur de plusieurs ordres de grandeur par rapport à une photodiode à base de silicium par exemple), ce qui a pour effet de dégrader le rapport signal à bruit, ou SNR (« Signal to Noise Ratio » en anglais) mesuré. Or, le SNR doit être important pour être capable de détecter des signaux faibles tels que ceux obtenus dans les applications de suivi de paramètres physiologiques et d'obtenir une mesure fiable et robuste.

Afin d'améliorer le SNR d'une OPD, le document *"*Vacuum-Processed Small Molecule Organic Photodetectors with Low Dark Current Density and Strong Response to Near-Infrared Wavelength" de C-C. Lee et al., Adv. Optical Mater. 2020, vol. 8, Issue 17, p. 2000519, propose l'utilisation de matériaux organiques à faible bruit. L'utilisation de tels matériaux est toutefois contraignante.

Le document *"*Organic narrowband near-infrared photodetectors based on intermolecular charge-transfer absorption" de Siegmund, B. et al., Nat. Commun 8, 15421 (2017) propose d'améliorer le SNR d'une OPD en réalisant, en son sein, une cavité optique pour emprisonner la lumière à détecter dans l'empilement de l'OPD et provoquer plusieurs allers et retours de la lumière à travers le matériau photosensible organique de l' OPD, et ainsi augmenter l'absorption lumineuse par le matériau photosensible pour, au final, augmenter le niveau de signal électrique de sortie obtenu tout en gardant le niveau de bruit identique. Cette solution est efficace pour une longueur d'onde donnée définie par l'épaisseur de la cavité optique. Toutefois, lorsque plusieurs longueurs d'onde sont destinées à être détectées par l'OPD ou que la longueur d'onde à détecter est susceptible de changer, la cavité résonnante n'apporte plus d'avantage.

Des problèmes analogues se retrouvent également dans d'autres domaines tels que celui des communications optiques, par exemple lorsqu'il s'agit d'optimiser le couplage entre un élément de guidage optique, par exemple un guide d'onde, et un composant électronique de conversion de lumière en un signal électrique.

### Résumé de l'invention

Il existe un besoin de proposer une solution palliant au moins une partie des inconvénients exposés ci-dessus.

Un mode de réalisation pallie tout ou partie de ces inconvénients et propose un dispositif photodétecteur comprenant au moins :
- un élément de guidage optique ;
- un composant électronique de conversion de lumière en un signal électrique, configuré pour détecter de la lumière au moins du côté d'une première face du composant électronique disposée en regard de l'élément de guidage optique et du côté d'une deuxième face du composant électronique opposée à la première face ;
- une couche réflectrice disposée du côté de la deuxième face du composant électronique ;
- un composant optique disposé entre la couche réflectrice et l'élément de guidage optique et au moins partiellement transparent à la lumière destinée à être détectée par le composant électronique ;
   et dans lequel la couche réflectrice forme au moins une surface réfléchissante conforme à une surface non plane du composant optique sur laquelle la couche réflectrice est disposée.

Selon un mode de réalisation particulier, le composant électronique comporte au moins une photodiode.

Selon un mode de réalisation particulier, le composant électronique comporte au moins une couche de matériau organique.

Selon un mode de réalisation particulier, le composant optique comporte au moins une partie concave.

Selon un mode de réalisation particulier, la surface réfléchissante forme au moins un miroir sphérique ou conique ou hyperbolique ou parabolique.

Selon un mode de réalisation particulier, la couche réflectrice comporte au moins une couche métallique et/ou au moins un miroir de Bragg.

Selon un mode de réalisation particulier, le dispositif photodétecteur comporte en outre au moins un substrat au moins partiellement transparent à la lumière destinée à être détectée par le composant électronique et disposé entre l'élément de guidage optique et le composant électronique.

Selon un mode de réalisation particulier, l'élément de guidage optique comporte au moins une microaiguille au moins partiellement transparente à la lumière destinée à être détectée par le composant électronique, ou au moins un guide d'onde.

Selon un mode de réalisation particulier, l'élément de guidage optique comporte une base au moins partiellement transparente à la lumière destinée à être détectée par le composant électronique et plusieurs microaiguilles au moins partiellement transparentes à la lumière destinée à être détectée par le composant électronique et comprenant chacune une première extrémité solidaire de la base, la base étant disposée entre le composant électronique et les microaiguilles.

Selon un mode de réalisation particulier, le dispositif photodétecteur comprend plusieurs composants électroniques distincts.

Selon un mode de réalisation particulier, chacun des composants électroniques est disposé à l'aplomb d'une des microaiguilles, ou chacun des composants électroniques est disposé à l'aplomb d'un groupe de microaiguilles, ou un groupe de composants électroniques est disposé à l'aplomb de chacune des microaiguilles, ou des composants électroniques sont disposés à l'aplomb d'espaces entre des microaiguilles.

Il est également proposé un dispositif de mesure de paramètre physiologique comprenant au moins un dispositif photodétecteur tel que précédemment décrit.

Il est également proposé un dispositif photovoltaïque comprenant au moins un dispositif photodétecteur tel que précédemment décrit.

Il est également proposé un procédé de réalisation d'un dispositif photodétecteur, comprenant au moins :
- réalisation d'au moins un élément de guidage optique ;
- réalisation d'au moins un composant électronique de conversion de lumière en un signal électrique, configuré pour détecter de la lumière au moins du côté d'une première face disposée en regard de l'élément de guidage optique et du côté d'une deuxième face opposée à la première face ;
- réalisation d'au moins une couche réflectrice disposée du côté de la deuxième face du composant électronique ;
- réalisation d'au moins un composant optique disposé entre la couche réflectrice et l'élément de guidage optique et au moins partiellement transparent à la lumière destinée à être détectée par le composant électronique ;
   et dans lequel la couche réflectrice est réalisée telle qu'elle forme au moins une surface réfléchissante conforme à une surface non plane du composant optique sur laquelle la couche réflectrice est disposée.

Selon un mode de réalisation particulier :
- le composant électronique et la couche réflectrice sont réalisés sur l'élément de guidage optique, ou
- le composant électronique et la couche réflectrice sont réalisés sur un substrat au moins partiellement transparent à la lumière destinée à être détectée par le composant électronique, le substrat étant solidarisé ensuite à l'élément de guidage optique.

### Brève description des dessins

Ces caractéristiques et avantages, ainsi que d'autres, seront exposés en détail dans la description suivante de modes et exemples de réalisation particuliers faite à titre non limitatif en relation avec les figures jointes parmi lesquelles :
- la figure 1 représente schématiquement un exemple de dispositif photodétecteur selon un mode de réalisation particulier ;
- la figure 2 représente des courbes montrant la puissance lumineuse reçue par un composant électronique de photoconversion configuré pour détecter de la lumière sur deux faces opposées, en fonction du rayon du composant électronique de photoconversion, dans un dispositif photodétecteur selon un mode de réalisation particulier ;
- la figure 3, la figure 4, la figure 5, la figure 6, la figure 7, la figure 8, la figure 9 et la figure 10 représentent des étapes d'un exemple de procédé de réalisation d'un dispositif photodétecteur selon un mode de réalisation particulier.

### Description des modes de réalisation

De mêmes éléments ont été désignés par de mêmes références dans les différentes figures. En particulier, les éléments structurels et/ou fonctionnels communs aux différents modes de réalisation peuvent présenter les mêmes références et peuvent disposer de propriétés structurelles, dimensionnelles et matérielles identiques.

Sur les figures, afin de faciliter leur lecture, les différents éléments et les différentes couches de matériaux ne sont pas nécessairement représentés à la même échelle les uns par rapport aux autres.

Par souci de clarté, seuls les étapes et éléments utiles à la compréhension des modes de réalisation décrits ont été représentés et sont détaillés.

Sauf précision contraire, lorsque l'on fait référence à deux éléments connectés entre eux, cela signifie directement connectés sans éléments intermédiaires autres que des conducteurs, et lorsque l'on fait référence à deux éléments reliés ou couplés (en anglais "coupled") entre eux, cela signifie que ces deux éléments peuvent être connectés ou être reliés par l'intermédiaire d'un ou plusieurs autres éléments.

Dans la description qui suit, lorsque l'on fait référence à des qualificatifs de position absolue, tels que les termes "avant", "arrière", "haut", "bas", "gauche", "droite", etc., ou relative, tels que les termes "dessus", "dessous", "supérieur", "inférieur", "latéral", etc., ou à des qualificatifs d'orientation, tels que les termes "horizontal", "vertical", etc., il est fait référence sauf précision contraire à l'orientation des figures. Toutefois, ces termes ne présument pas de la position et de l'orientation réelles du dispositif lors de son utilisation.

Sauf précision contraire, les expressions "environ", "approximativement", "sensiblement", et "de l'ordre de" signifient à 10 % près, de préférence à 5 % près. En outre, sauf indication contraire, les gammes de valeurs données incluent les bornes de ces gammes.

Dans tout le document, l'expression « au moins partiellement transparent » est utilisée pour caractériser le fait qu'un élément puisse être traversé par au moins une partie (par exemple au moins 40 % ou au moins 50 % ou au moins 70 % ou au moins 90 %) de la lumière reçue en entrée de cet élément.

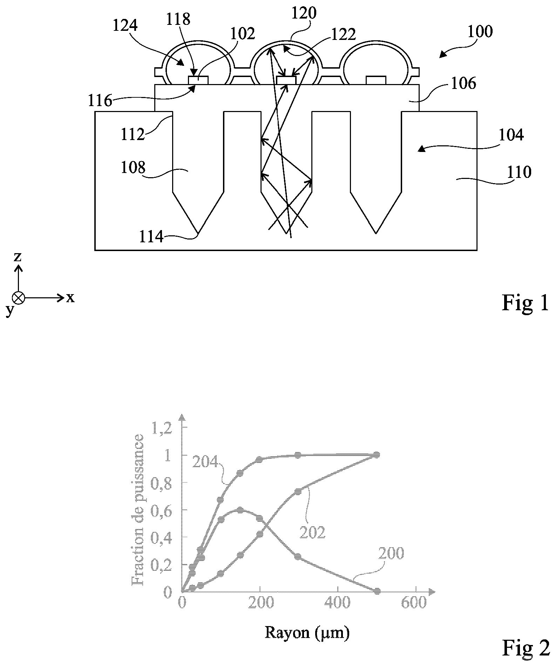

Un exemple de dispositif photodétecteur 100 selon un mode de réalisation particulier est décrit ci-dessous en lien avec la figure 1.

Dans cet exemple de réalisation, le dispositif 100 correspond à un dispositif de mesure de paramètre physiologique doté d'une partie formée de microaiguilles destinées à être insérées dans des tissus, à travers la surface extérieure de la peau.

Le dispositif 100 comporte au moins un composant électronique 102 de conversion de lumière en un signal électrique, c'est-à-dire un composant électronique de photoconversion ou de photodétection. Dans l'exemple de réalisation décrit, le dispositif 100 comporte plusieurs composants électroniques 102 distincts et espacés les uns des autres. Néanmoins, le dispositif 100 peut comporter un unique composant électronique 102.

Dans l'exemple de réalisation décrit, chacun des composants électroniques 102 comporte au moins une photodiode. Plus particulièrement, dans l'exemple décrit, chaque composant électronique 102 correspond à une photodiode. En outre, dans cet exemple, les composants électroniques 102 sont de nature organique, c'est-à-dire comportent un ou plusieurs matériaux organiques, et correspondent à des OPDs.

Dans l'exemple décrit, chacun des composants électroniques 102 comporte des première et deuxième électrodes, l'une correspondant à l'anode et l'autre correspondant à la cathode de la photodiode formée par le composant électronique 102, à base d'au moins un matériau électriquement conducteur. Les électrodes sont au moins partiellement transparentes (et de préférence totalement ou quasi-totalement transparentes) à au moins une lumière destinée à être détectée par le composant électronique 102. De plus, chacun des composants électroniques 102 comporte au moins une couche de photodétection, ou couche photosensible, comprenant au moins un matériau semi-conducteur organique et disposée entre les première et deuxième électrodes. L'épaisseur (dimension parallèle à l'axe Z sur la figure 1) de chaque composant électronique 102 est par exemple comprise entre 50 nm et 500 nm. A titre d'exemple, les électrodes de chacun des composants électroniques 102 peuvent comporter au moins l'un des matériaux suivants : Al, Ag, ITO, SnO₂, etc. La couche de photodétection de chaque composant électronique 102 peut comporter, en fonction de la ou des longueurs d'onde destinées à être détectées, au moins l'un des types de matériaux suivants : CuPc (phtalocyanine de cuivre), ZnPc (phtalocyanine de zinc), C₆₀, ClAlPc (phtalocyanine de chloroaluminium), PCBM, PbPc (phtalocyanine de plomb), etc.

Le dispositif 100 comporte en outre au moins un élément de guidage optique 104. Dans l'exemple de réalisation décrit, l'élément de guidage optique 104 comporte une base 106 au moins partiellement transparente (et de préférence totalement ou quasi-totalement transparente) à la lumière destinée à être détectée par les composants électroniques 102, ainsi que plusieurs microaiguilles 108 également au moins partiellement transparentes (et de préférence totalement ou quasi-totalement transparentes) à la lumière destinée à être détectée par les composants électroniques 102. Les microaiguilles 108 sont ici destinées à être insérées dans des tissus 110 correspondant à des couches superficielles de la peau. Chacune des microaiguilles 108 comporte une première extrémité 112 solidaire de la base 106 et une deuxième extrémité 114 en forme de pointe. Plus particulièrement, dans l'exemple décrit, chaque microaiguille 108 comporte une partie cylindrique s'étendant depuis la première extrémité 112 et se prolongeant par une partie conique jusqu'à la deuxième extrémité 114. En variante, des formes de microaiguilles 108 différentes de celle décrite ci-dessus sont possibles. Par exemple, la section des microaiguilles 108 peut être d'une forme autre qu'un disque. En outre, des formes autres qu'une pointe sont envisageables, par exemple pour orienter l'élément de guidage optique 104 ou le coupler avec un diffuseur local disposé au bout des microaiguilles 108.

Dans l'exemple de réalisation décrit, les microaiguilles 108 sont configurées pour guider la lumière diffusée depuis les tissus 110 jusqu'au niveau de la surface de la base 106 sur laquelle sont disposées les composants électroniques 102. La forme conique des pointes des microaiguilles 108 permet de bien faire pénétrer les microaiguilles 108 dans la peau et également d'assurer une bonne collection de la lumière guidée dans la partie cylindrique des microaiguilles 108. Dans l'exemple décrit, le guidage lumineux au sein des microaiguilles 108 est réalisé grâce à la différence d'indice de réfraction entre le matériau des microaiguilles 108 et les tissus 110. A titre d'exemple, la hauteur de chacune des microaiguilles 108 (dimension parallèle à l'axe Z sur la figure 1) peut être comprise entre 100 µm et 3 mm. La section de la partie cylindrique de chacune des microaiguilles 108 a par exemple, dans un plan perpendiculaire à leur hauteur (plan parallèle au plan (X,Y) sur l'exemple de la figure 1), un diamètre compris entre 50 µm et 900 µm. Le pas des microaiguilles 108, c'est-à-dire la distance séparant les axes de révolution de deux microaiguilles 108 voisines, peut être compris entre environ 100 µm et plusieurs millimètres, ou être supérieur ou égal à 500 µm. Selon un exemple de réalisation, les microaiguilles 108 peuvent comporter un matériau biocompatible tel qu'un polymère, par exemple du polyméthacrylate de méthyle ou PMMA, ou du PLGA (poly (acide lactique-co-glycolique)).

Lorsque le dispositif 100 est destiné à des applications autres que la mesure de paramètres physiologiques par photodétection de lumière se propageant dans la peau, l'élément de guidage optique 104 peut comporter la base 106 à laquelle sont couplés optiquement un ou plusieurs éléments au moins partiellement transparents (et de préférence totalement ou quasi-totalement transparents) à la lumière destinée à être détectée par les composants électroniques 102, ce ou ces éléments pouvant être différents des microaiguilles 108.

Ainsi, l'élément de guidage optique 104 peut comporter par exemple au moins une microaiguille, ou au moins un guide d'onde (exemple d'une application autre que la mesure de paramètres physiologiques) ou d'autres types d'élément selon l'application envisagée (par exemple le domaine du photovoltaïque), et l'élément de guidage optique 104 peut comporter ou non la base 106.

Chacun des composants électroniques 102 est configuré pour détecter de la lumière au moins du côté d'une première face 116 disposée en regard de l'élément de guidage optique 104 et également du côté d'une deuxième face 118 opposée à la première face 116. Cette détection lumineuse du côté de chacune des faces 116, 118 est due, dans cet exemple de réalisation, au fait que les composants électroniques 102 correspondent à des OPD dont la ou les couches organiques de photodétection détectent de la lumière du côté de chacune des électrodes (première électrode disposée du côté de la première face 116 et deuxième électrode disposée du côté de la deuxième face 118). En variante, cette détection lumineuse depuis les deux faces 116, 118 des composants électroniques 102 peut être obtenue en utilisant d'autres types de composants électroniques 102 configurés pour détecter de la lumière depuis deux côtés, ou faces, opposés.

Dans l'exemple de réalisation décrit, la première face 116 de chacun des composants électroniques 102 est disposée directement contre l'élément de guidage optique 104, et plus particulièrement contre la base 106 de l'élément de guidage optique 104. En variante, il est possible qu'au moins un élément au moins partiellement transparent, ou de préférence totalement ou quasi-totalement transparent, à la lumière destinée à être détectée par les composants électroniques 102, par exemple un substrat de verre ou de tout autre matériau transparent ou semi-transparent, soit interposé entre les composants électroniques 102 et l'élément de guidage optique 104, comme par exemple des bases coniques sur lesquelles sont disposées les microaiguilles, et avec une éventuelle semelle sur laquelle reposent les bases coniques.

Le dispositif 100 comporte en outre au moins une couche réflectrice 120 disposée du côté des deuxièmes faces 118 des composants électroniques 102 et formant une surface réfléchissante 122 disposée en regard des composants électroniques 102. Cette surface 122 est qualifiée de réfléchissante du fait qu'elle est configurée pour réfléchir au moins une partie et, de préférence, la totalité ou la quasi-totalité de la lumière transmise depuis l'élément de guidage optique 104 et qui n'a pas été absorbée par les premières faces 116 des composants électroniques 102, pour augmenter la quantité totale de lumière absorbée par les composants électroniques 102. La couche réflectrice 120 comporte par exemple au moins un métal tel que de l'argent ou de l'aluminium. L'épaisseur de la couche réflectrice 120 est par exemple comprise entre 50 nm et 500 nm.

En variante, la couche réflectrice 120 peut comporter au moins un miroir de Bragg configuré pour réfléchir la ou les longueurs d'onde d'intérêt destinées à être détectées par les composants électroniques 102.

Dans tous les cas, les propriétés de la couche réflectrice 120 (matériau(x) utilisé(s), épaisseur, forme, etc.) peuvent être telles que de la surface réfléchissante 122 réfléchisse le plus de lumière possible afin d'avoir une perte lumineuse qui soit la plus faible possible au niveau de cette couche réflectrice 120.

Le dispositif 100 comporte en outre au moins un composant optique 124 disposé entre la couche réflectrice 120 et l'élément de guidage optique 104, et plus particulièrement entre chacun des composants électroniques 102 et la couche réflectrice 120. Sur l'exemple de la figure 1, le dispositif 100 comporte plusieurs composants optiques 124 chacun disposé à l'aplomb de l'une des microaiguilles 108 et également disposé entre l'un des composants électroniques 102 et la couche réflectrice 120. Le pas (distance entre les centres de deux composants optiques 124 voisins) avec lequel les composants optiques 124 sont réalisés peut être égal à celui des microaiguilles 108. Les composants optiques 124 sont au moins partiellement transparents, et de préférence totalement ou quasi-totalement transparents, à la lumière destinée à être détectée par les composants électroniques 102. Selon un exemple de réalisation, les composants optiques 124 comportent un polymère de type résine (par exemple du PMMA ou du PLGA) ou un oxyde tel que du SiO₂ ou bien du SiN ou tout autre matériau adapté. En outre, l'épaisseur de chacun des composants optiques 124 (c'est-à-dire leur dimension parallèle à l'axe Z sur l'exemple de la figure 1) est par exemple comprise entre 50 µm et 2 mm, ou supérieur ou égal à 100 µm.

La couche réflectrice 120 est disposée sur les composants optiques 124 telle que la surface réfléchissante 122 soit conforme à une surface non plane des composants optiques 124 et réalise ainsi une réflexion lumineuse selon une directivité et/ou une focalisation souhaitée définie par la forme de la surface non plane des composants optiques 124. Ainsi, la géométrie de la surface réfléchissante 122 en regard de chaque microaiguille 108 dépend de celle de la surface non plane de chaque composant optique 124. Dans l'exemple de réalisation décrit, chaque composant optique 124 forme une surface concave sur laquelle la couche réflectrice 120 est disposée, cette forme correspondant à celle de la surface réfléchissante 122. Par exemple, les composants optiques 124 peuvent être tels que la surface réfléchissante 122 forme, en regard de chaque microaiguille 108, au moins un miroir sphérique, ou calotte sphérique, ou conique, ou hyperbolique, ou avantageusement parabolique. D'autres formes sont envisageables : coin de cube, ellipse, etc. En variante, chaque composant optique 124 peut avoir une autre forme non plane adaptée pour que la surface réfléchissante 122 réalise une réflexion lumineuse souhaitée en direction des composants électroniques 102. Par exemple, les composants optiques 124 peuvent être tels que, combinés à la surface réfléchissante 122, ils permettent d'augmenter localement la directivité de la lumière pour la renvoyer avec un angle adéquat vers les composants électroniques 102.

Par exemple, lorsque le dispositif 100 est utilisé pour le domaine photovoltaïque, avoir une surface réfléchissante 122 formant un miroir parabolique permet de couvrir une gamme spectrale bien plus large que lorsque des microlentilles sont utilisées (limitées par les propriétés de réflexion des matériaux utilisés). Les avantages sont obtenus pour toutes les longueurs d'onde.

Ainsi, les composants optiques 124 combinés à la surface réfléchissante 122 peuvent être configurés pour focaliser la lumière du côté de la deuxième face 118 de chacun des composants électroniques 102.

Dans le dispositif 100, afin de limiter la diaphonie, ou « crosstalk » en anglais, entre les composants électroniques 102, il est possible de réduire le plus possible l'épaisseur de la base 106, et plus généralement de réduire la distance entre les composants électroniques 102 et les premières extrémités 112 des microaiguilles 108 pour éviter que de la lumière issue d'une des microaiguilles 108 soit détectée par un composant électronique 102 différent de celui disposé à l'aplomb de cette microaiguille.

En variante de l'exemple de réalisation décrit ci-dessus, l'élément de guidage optique 104 peut correspondre à un guide d'onde. Le dispositif 100 selon une telle variante peut par exemple être utilisé dans le domaine des communications optiques afin d'optimiser le couplage optique entre les composants électroniques 102 et le guide d'onde correspondant à l'élément de guidage optique 104.

Des courbes représentées sur la figure 2 montrent la fraction de puissance lumineuse issue d'une des microaiguilles 108 et reçue par l'un des composants électroniques 102 disposé à l'aplomb de cette microaiguille 108, en fonction du rayon (en microns) du composant électronique 102 (en considérant ici que le composant électronique 102 a, dans un plan perpendiculaire à l'axe de révolution de la microaiguille 108, une section en forme de disque). Sur la figure 2, la fraction de puissance reçue est définie comme étant le rapport entre la puissance lumineuse détectée et la puissance lumineuse totale émise initialement depuis la microaiguille 108. La courbe 200 représente la fraction de puissance lumineuse reçue à travers la deuxième face 118 du composant électronique 102, la courbe 202 représente la fraction de puissance lumineuse reçue à travers la première face 116 du composant électronique 102, et la courbe 204 représente la somme des fractions de puissance lumineuse reçue à travers les deux faces 116, 118 du composant électronique 102. Ces valeurs sont obtenues pour :
- une microaiguille 108 de diamètre égal à 400 µm depuis laquelle un flux lumineux est issu avec un demi-angle d'ouverture de 25° ;
- une surface réflectrice 122 de forme conique parabolique de rayon égal à 1 mm, de hauteur (dimension parallèle à l'axe de révolution de la microaiguille 108) égale à 0,312 mm et de focal égale à 0,2 mm, le composant électronique 102 étant centré sur le point focal de la surface réfléchissante 122.

Dans ce cas de figure, lorsque le rayon du composant électronique 102 est égal à 500 µm, 100 % de la lumière est détectée par la première face 116 du composant électronique 102. En réduisant le diamètre du composant électronique 102, 100 % de la lumière est toujours détectée par le composant électronique 102 lorsque le rayon du composant électronique 102 est compris entre 500 µm et 220 µm, grâce à la réflexion de la lumière sur la surface réfléchissante 122 et sa déviation vers la deuxième face 118 du composant électronique 102. Ces courbes montrent qu'il est possible de réduire fortement les dimensions du composant électronique 102, et donc, dans le cas d'un composant électronique 102 correspondant à une OPD, de réduire fortement son courant d'obscurité et ainsi augmenter fortement son SNR, tout en conservant un important taux de détection de la lumière.

En variante, il est possible qu'un ou plusieurs composants électroniques 102 soient disposés non pas en regard de la ou des microaiguilles 108, mais à côté ou entre celles-ci. Ainsi, il est par exemple possible de dissocier l'information provenant de la surface des tissus 110 de celle provenant de l'intérieur des tissus 110, et mesurer ainsi l'information potentielle dans l'espace inter-microaiguilles. Dans une telle variante, le ou les composants électroniques 102 peuvent être disposés sur la base 106. De plus, dans une telle variante, le ou les composants électroniques 102 peuvent correspondre à une ou des OPDs structurées.

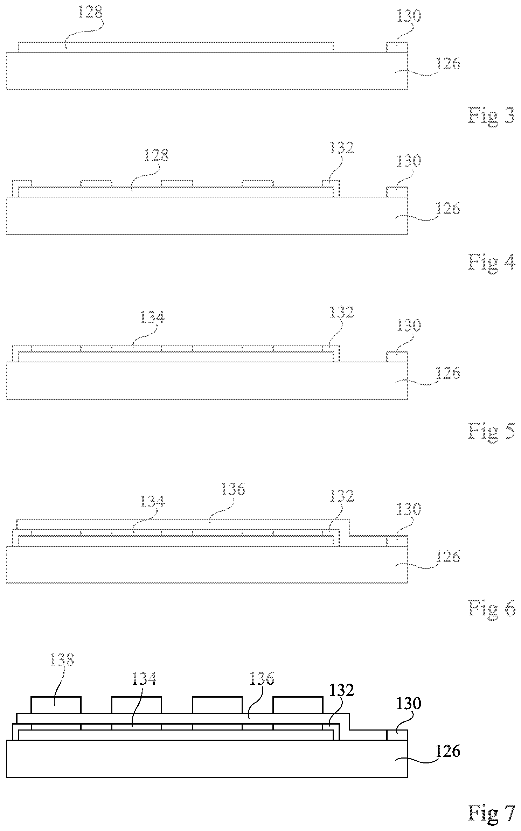

Un exemple de procédé de réalisation du dispositif 100 est décrit ci-dessous en lien avec les figures 3 à 10.

Dans cet exemple, les composants électroniques 102, la couche réflectrice 120 et les composants optiques 124 sont réalisés sur un substrat 126 au moins partiellement transparent, et de préférence totalement ou quasi-totalement transparent, à la lumière destinée à être détectée par les composants électroniques 102. L'épaisseur du substrat 126 est par exemple égale à quelques centaines de microns. Par exemple, le substrat 126 peut comporter du verre.

En variante, il est possible que les composants électroniques 102, la couche réflectrice 120 et les composants optiques 124 soient réalisés directement sur l'élément de guidage optique 104, comme c'est le cas sur l'exemple de la figure 1.

Dans l'exemple de réalisation décrit, les composants électroniques 102 correspondent à des OPD. Ainsi, dans cet exemple, une première électrode 128 transparente ou semi-transparente, c'est-à-dire apte à laisser passer au moins une partie de la lumière destinée à être détectée par les composants électroniques 102, est réalisée sur le substrat 126. La première électrode 128 correspond par exemple à l'anode des composants électroniques 102. Au moins un plot de contact 130 auquel une deuxième électrode des composants électroniques 102 est destinée à être couplée électriquement est également réalisé sur le substrat 126, à côté de la première électrode 128 (voir figure 3).

Dans l'exemple décrit, la première électrode 128 est commune aux différents composants électroniques 102. En variante, il est possible que chaque composant électronique 102 comporte une première électrode 128 distincte et isolée électriquement des premières électrodes 128 des autres composants électroniques 102, ou bien que plusieurs premières électrodes 128 distinctes et isolées électriquement les unes des autres soient réalisées sur le substrat 126, chacune d'elles étant couplée à plusieurs composants électroniques 102.

Des portions isolantes 132, comprenant par exemple de la résine, sont ensuite formées, par exemple par dépôt, en périphérie de la première électrode 128 et entre des emplacements des futures régions actives de photodétection des composants électroniques 102, c'est-à-dire entre les emplacements où seront disposées les portions de la ou des couches destinées à assurer une détection de lumière (voir figure 4). Les portions isolantes 132 disposées sur les bords de la première électrode 128 sont destinées à isoler électriquement la première électrode 128 vis-à-vis de la liaison électrique qui sera réalisée entre la deuxième électrode des composants électroniques 102 et le plot de contact 130.

Une ou plusieurs couches de détection lumineuse 134, comprenant ici au moins un matériau organique, sont ensuite déposées sur la première électrode 128, entre les portions isolantes 132 (voir figure 5). Ce dépôt est par exemple mis en œuvre à travers un pochoir pour localiser le dépôt de cette ou ces couches de détection lumineuse 134 aux emplacements souhaités, entre les portions isolantes 132.

Une deuxième électrode 136 transparente ou semi-transparente est ensuite réalisée sur la ou les couches de détection lumineuse 134 et les portions isolantes 132. Cette deuxième électrode 136 correspond par exemple à la cathode des composants électroniques 102. Une partie de cette deuxième électrode 136 est déposée sur au moins une des portions isolantes 132 disposée sur l'un des bords de la première électrode 128 et sur une partie du substrat 126 de manière à être en contact avec le plot de contact 130 (voir figure 6).

Dans l'exemple décrit, la deuxième électrode 136 est commune aux différents composants électroniques 102. En variante, il est possible que chaque composant électronique 102 comporte une deuxième électrode 136 distincte et isolée électriquement des deuxièmes électrodes 136 des autres composants électroniques 102, ou bien que plusieurs deuxièmes électrodes 136 distinctes et isolées électriquement les unes des autres soient réalisées, chacune d'elles étant couplée à plusieurs composants électroniques 102.

A ce stade du procédé, la réalisation des composants électroniques 102 est achevée.

Bien que non visible, au moins une couche d'encapsulation transparente ou semi-transparente peut être déposée ensuite sur les composants électroniques 102.

Les composants optiques 124 sont ensuite réalisés sur les composants électroniques 102. Dans l'exemple de réalisation décrit, des plots 138 du ou des matériaux destinés à former les composants optiques 124, par exemple des plots de résine transparente ou semi-transparente, sont réalisés par exemple par dépôt au-dessus de la deuxième électrode 136 (voir figure 7). En présence d'une couche d'encapsulation recouvrant les composants électroniques 102, les plots 138 sont réalisés sur cette couche d'encapsulation.

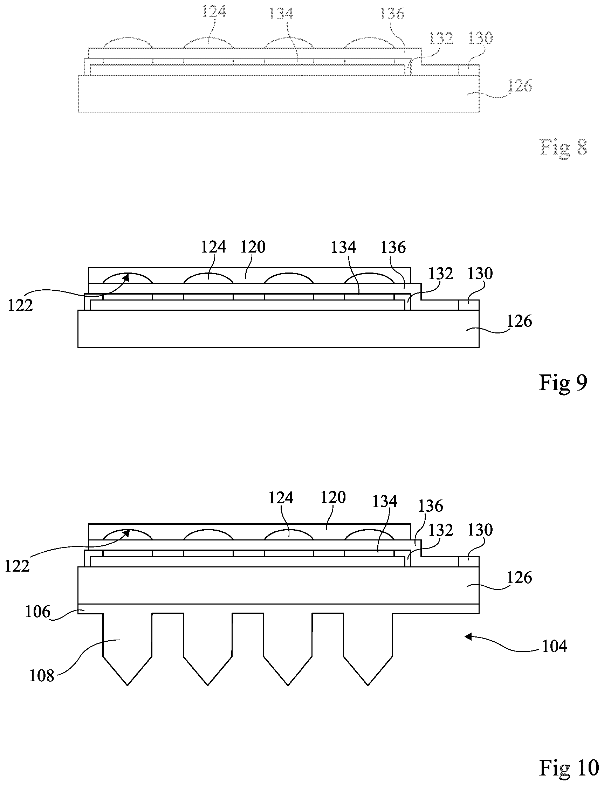

Une étape de fluage peut ensuite être mise en œuvre pour donner aux plots 138 la forme souhaitée et former ainsi les composants optiques 124 (voir figure 8).

La couche réflectrice 120 est ensuite réalisée, par exemple par dépôt, sur les composants optiques 124 et sur les parties de la deuxième électrode 136 non recouvertes par les composants optiques 124 (voir figure 9).

Le dispositif 100 est achevé en reportant la structure réalisée sur l'élément de guidage 104 comprenant, dans l'exemple de réalisation décrit, la base 106 et les microaiguilles 108. Ce report correspond, dans l'exemple décrit, à une solidarisation du substrat 126 contre la base 106 (voir figure 10).

Dans une variante, les composants électroniques 102 peuvent être réalisés tels qu'ils soient configurés pour détecter des lumières de longueurs d'onde différentes. Dans ce cas, la ou les couches de détection lumineuse 134 déposées sont par exemple différentes en fonction de la ou des longueurs destinées à être détectées.

Dans les exemples de réalisation précédemment décrits, le dispositif 100 comporte plusieurs composants électroniques 102 chacun disposé à l'aplomb de l'une des microaiguilles 108. En variante, le dispositif 100 peut comporter un seul composant électronique 102, correspondant par exemple à une seule photodiode, disposé à l'aplomb de l'ensemble des microaiguilles 108. Selon une autre variante, le dispositif 100 peut comporter plusieurs composants électroniques 102 tels que chacun d'eux soit disposé à l'aplomb d'un groupe de microaiguilles 108. Selon une autre variante, le dispositif 100 peut comporter plusieurs composants électroniques 102 tels qu'un groupe de composants électroniques 102 soit disposé à l'aplomb de chacune des microaiguilles 108.

Dans l'exemple de réalisation précédemment décrit, le dispositif 100 correspond à un dispositif utilisé dans le domaine de la santé pour suivre, ou mesurer, des paramètres physiologiques et/ou thérapeutiques. Dans le dispositif 100, la lumière est une modalité de mesure utilisée pour déterminer et suivre des paramètres physiologiques d'intérêt tels que le rythme cardiaque, le taux d'oxygénation du sang, la glycémie, la SpO2, ou bien la SaO2. La lumière guidée dans l'élément de guidage optique 104 et détectée par les composants électroniques 102 correspond à de la lumière se propageant dans les tissus 110 et qui est issue par exemple d'une source de lumière extérieure aux tissus 110. La lumière guidée dans l'élément de guidage optique 104 a, préalablement à son entrée dans l'élément de guidage optique 104, interagi avec les tissus d'intérêts qui porte l'information permettant de remonter aux paramètres physiologiques ciblés.

Quelle que soit l'application visée, le dispositif 100 permet de maximiser la collecte des photons sur le ou les composants électroniques 102 tout en gardant, lorsque les composants électroniques 102 correspondent à des photodiodes, une surface de détection réduite permettant de minimiser le courant d'obscurité et donc d'augmenter le SNR des photodiodes afin de rendre la mesure plus fiable et plus robuste, et cela nonobstant la faible force du signal optique capté en raison du fort taux d'absorption dans la peau et de la diffusion de la lumière dans les tissus 110.

A titre d'exemple, comparativement à un dispositif photodétecteur qui ne serait pas muni de la surface réfléchissante 122 permettant de renvoyer la lumière vers la deuxième face 118 des composants électroniques 102, il est possible, avec le dispositif 100, de réduire la surface active de photodétection des composants électroniques 102 par exemple d'un facteur 5,2, ce qui réduit le courant d'obscurité d'autant, sans réduire la fraction de puissance lumineuse détectée par les composants électroniques 102.

Ces avantages sont également obtenus quelle que soit la longueur de la lumière détectée, et également lorsque la lumière est polychromatique. De plus, ces avantages sont obtenus sans contraintes particulières sur les matériaux organiques pouvant être utilisés pour la réalisation des composants électroniques 102, et le dispositif 100 est compatible avec des matériaux optimisés pour la photodétection souhaitée.

Le dispositif 100 permet d'optimiser la détection de la lumière guidée par l'élément de guidage optique 104 grâce à l'utilisation judicieuse de la surface réfléchissante 122 non plane et qui, combinée à un ou plusieurs composants électroniques 102 photodétecteurs détectant de la lumière à la fois du côté de l'élément de guidage optique 104 et du côté de la surface réfléchissante 122, permet d'augmenter la quantité de lumière envoyée vers le ou les composants électroniques 102 étant donné que la lumière atteignant la surface réfléchissante 122 est récupérée et réfléchie en direction du ou des composants électroniques 102 grâce aux propriétés de réflexion et de focalisation de la lumière de la surface réfléchissante 122.

Dans cet exemple de dispositif 100, la lumière provenant des tissus 110 est guidée par les microaiguilles 108. Une partie de cette lumière peut être absorbée par la première face 116 des composants électroniques 102 alors que celle qui ne l'est pas est réfléchie par la surface réfléchissante 122 et renvoyée vers la deuxième face 118 des composants 102 pour y être détectée.

Le fait d'avoir des composants électroniques 102 correspondant à des photodiodes permet de bénéficier des propriétés d'intégrabilité de celles-ci. Par exemple, les OPD ont pour avantage d'être robuste aux étapes mises en œuvre pour la réalisation des composants optiques 124.

Le dispositif 100 peut être utilisé pour d'autres domaines d'application que ceux précédemment décrits, par exemple au sein de « smart pixels », ou pixels intelligents, dans les écrans afin d'optimiser le SNR de ces pixels, ou bien dans tout domaine demandant la réalisation d'une photodiode ou d'un photodétecteur et notamment quand le signal reçu par la photodiode est faible, ou encore dans le domaine photovoltaïque.

Divers exemples de réalisation et variantes ont été décrits. La personne du métier comprendra que certaines caractéristiques de ces divers exemples de réalisation et variantes pourraient être combinées, et d'autres variantes apparaîtront à la personne du métier.

Enfin, la mise en œuvre pratique des modes de réalisation et variantes décrits est à la portée de la personne du métier à partir des indications fonctionnelles données ci-dessus. Par exemple, la nature précise des étapes de dépôt et de gravure mises en œuvre peut être choisie en fonction notamment du ou des matériaux à déposer ou à graver, ainsi que des épaisseurs des matériaux à déposer ou à graver.

## Revendications

1. Dispositif photodétecteur (100) comprenant au moins :
- un élément de guidage optique (104) ;
- un composant électronique (102) de conversion de lumière en un signal électrique, configuré pour détecter de la lumière au moins du côté d'une première face (116) du composant électronique (102) disposée en regard de l'élément de guidage optique (104) et du côté d'une deuxième face (118) du composant électronique (102) opposée à la première face (116) ;
- une couche réflectrice (120) disposée du côté de la deuxième face (118) du composant électronique (102) ;
- un composant optique (124) disposé entre la couche réflectrice (120) et l'élément de guidage optique (104) et au moins partiellement transparent à la lumière destinée à être détectée par le composant électronique (102) ;
et dans lequel la couche réflectrice (120) forme au moins une surface réfléchissante (122) conforme à une surface non plane du composant optique (124) sur laquelle la couche réflectrice (120) est disposée.

2. Dispositif photodétecteur (100) selon la revendication **1,** dans lequel le composant électronique (102) comporte au moins une photodiode.

3. Dispositif photodétecteur (100) selon l'une quelconque des revendications précédentes, dans lequel le composant électronique (102) comporte au moins une couche de matériau organique (134).

4. Dispositif photodétecteur (100) selon l'une quelconque des revendications précédentes, dans lequel le composant optique (124) comporte au moins une partie concave.

5. Dispositif photodétecteur (100) selon l'une quelconque des revendications précédentes, dans lequel la surface réfléchissante (122) forme au moins un miroir sphérique ou conique ou hyperbolique ou parabolique.

6. Dispositif photodétecteur (100) selon l'une quelconque des revendications précédentes, dans lequel la couche réflectrice (120) comporte au moins une couche métallique et/ou au moins un miroir de Bragg.

7. Dispositif photodétecteur (100) selon l'une quelconque des revendications précédentes, comportant en outre au moins un substrat (126) au moins partiellement transparent à la lumière destinée à être détectée par le composant électronique (102) et disposé entre l'élément de guidage optique (104) et le composant électronique (102).

8. Dispositif photodétecteur (100) selon l'une quelconque des revendications précédentes, dans lequel l'élément de guidage optique (104) comporte au moins une microaiguille (108) au moins partiellement transparente à la lumière destinée à être détectée par le composant électronique (102), ou au moins un guide d'onde.

9. Dispositif photodétecteur (100) selon la revendication 8, dans lequel l'élément de guidage optique (104) comporte une base (106) au moins partiellement transparente à la lumière destinée à être détectée par le composant électronique (102) et plusieurs microaiguilles (108) au moins partiellement transparentes à la lumière destinée à être détectée par le composant électronique (102) et comprenant chacune une première extrémité (112) solidaire de la base (106), la base (106) étant disposée entre le composant électronique (102) et les microaiguilles (108).

10. Dispositif photodétecteur (100) selon l'une des revendications précédentes, comprenant plusieurs composants électroniques (102) distincts.

11. Dispositif photodétecteur (100) selon les revendications 9 et 10, dans lequel chacun des composants électroniques (102) est disposé à l'aplomb d'une des microaiguilles (108), ou dans lequel chacun des composants électroniques (102) est disposé à l'aplomb d'un groupe de microaiguilles (108), ou dans lequel un groupe de composants électroniques (102) est disposé à l'aplomb de chacune des microaiguilles (108), ou dans lequel des composants électroniques (102) sont disposés à l'aplomb d'espaces entre des microaiguilles (108).

12. Dispositif de mesure de paramètre physiologique comprenant au moins un dispositif photodétecteur (100) selon l'une quelconque des revendications précédentes.

13. Dispositif photovoltaïque comprenant au moins un dispositif photodétecteur (100) selon l'une quelconque des revendications 1 à 11.

14. Procédé de réalisation d'un dispositif photodétecteur (100), comprenant au moins :
- réalisation d'au moins un élément de guidage optique (104) ;
- réalisation d'au moins un composant électronique (102) de conversion de lumière en un signal électrique, configuré pour détecter de la lumière au moins du côté d'une première face (116) disposée en regard de l'élément de guidage optique (104) et du côté d'une deuxième face (118) opposée à la première face (116) ;
- réalisation d'au moins une couche réflectrice (120) disposée du côté de la deuxième face (118) du composant électronique (102) ;
- réalisation d'au moins un composant optique (124) disposé entre la couche réflectrice (120) et l'élément de guidage optique (124) et au moins partiellement transparent à la lumière destinée à être détectée par le composant électronique (102) ;
et dans lequel la couche réflectrice (120) est réalisée telle qu'elle forme au moins une surface réfléchissante (122) conforme à une surface non plane du composant optique (124) sur laquelle la couche réflectrice (120) est disposée.

15. Procédé de réalisation selon la revendication 14, dans lequel :
- le composant électronique (102) et la couche réflectrice (120) sont réalisés sur l'élément de guidage optique (104), ou
- le composant électronique (102) et la couche réflectrice (120) sont réalisés sur un substrat (126) au moins partiellement transparent à la lumière destinée à être détectée par le composant électronique (102), le substrat (126) étant solidarisé ensuite à l'élément de guidage optique (104).
